# EUROPEAN PATENT SPECIFICATION

(11) **EP 4 405 038 B1**
(45) Date of publication and mention of the grant of the patent: **26.08.2026**
(21) Application number: 21798533.2
(22) Date of filing: 22.09.2021
(51) Int. Cl.: A61N 5/10

(54) **PARTICLE THERAPY USING TEMPORO-SPATIAL DOSE HETEROGENEITIES**
PARTIKELTHERAPIE MIT TEMPORO-RÄUMLICHEN HETEROGENITÄTEN DER DOSIS
THÉRAPIE PAR DES PARTICULES UTILISANT DES HÉTÉROGÉNÉITÉS TEMPORO-SPATIALES DE DOSE

(43) Date of publication of application: 31.07.2024
(73) Proprietor: Elekta, Inc., Atlanta, GA 30346 (US)
(72) Inventor: SOUKUP, Martin, Atlanta, GA 30346 (US); TSAI, Kun-Yu, Atlanta, GA 30346 (US)
(74) Representative: Hamer, Thomas Daniel
(86) International application number: PCT/US2021/071549
(87) International publication number: WO 2023/048750

(56) References cited:
- US-A1- 2010 219 356
- US-A1- 2019 022 411
- MEIN STEWART ET AL: "Spot-Scanning Hadron Arc (SHArc) Therapy: A Study With Light and Heavy Ions", ADVANCES IN RADIATION ONCOLOGY, vol. 6, no. 3, 1 May 2021 (2021-05-01), pages 100661, XP055927805, ISSN: 2452-1094, DOI: 10.1016/j.adro.2021.100661

## Description

### BACKGROUND

Radiation therapy or radiotherapy has been a well-known procedure for cancer treatment. It uses high doses of radiation to kill cancer cells and shrink tumors. With the development of technology, a broad range of radiation energies have been used in clinical treatment. Typically, these include photon, electron and high energy particle therapy. High energy particle therapy, due to its advantage of providing superior dose distribution with minimal exit dose compared to other forms of radiation therapy, has been growing rapidly in recent decades. For example, Mein (Mein S, Tessonnier T, Kopp B, Harrabi S, Abdollahi A, Debus J, Haberer T, Mairani A. Spot-Scanning Hadron Arc (SHAre) Therapy: A Study With Light and Heavy Ions. Adv Radiat Oncol. 2021 Feb 4;6(3):100661) discusses a spot-scanning hadron arc radiotherapy, wherein reduction of toxicity in normal tissues, i.e. protecting healthy tissue is envisaged.

### BRIEF DESCRIPTION OF THE DRAWINGS

FIG. 1 illustrates generally radiation dose depths in human tissue for various types of particles, in accordance with an embodiment.
FIG. 2 illustrates generally pencil beam scanning of an irregular shape volume from distal edge to proximal edge, in accordance with an embodiment.
FIG. 3 illustrates generally a diagram of an active scanning proton beam delivery system, in accordance with an embodiment.
FIG. 4 illustrates a diagrammatic representation of a particle arc therapy, in accordance with an embodiment.
FIG. 5 illustrates a particle treatment planning system, in accordance with an embodiment.
FIG. 6 illustrates an example particle arc trajectory, in accordance with an embodiment.
FIG. 7 illustrates an extended particle treatment planning workflow for optimizing dose heterogeneity effects, in accordance with an embodiment.
FIGS. 8A-8C illustrate a high dose region, in accordance with an embodiment.
FIG. 9 illustrates an example particle arc trajectory satisfying dose heterogeneity by avoiding high dose region overlapped outside the target, in accordance with an embodiment.
FIGS. 10A-10D illustrate examples of different spot trajectory on single energy layer, in accordance with an embodiment.
FIGS. 11A-11B illustrate examples of spot trajectory after weight optimization, in accordance with an embodiment.
FIGS. 12A-12B illustrate example FLASH condition optimization results, in accordance with an embodiment.
FIG. 13 illustrates an example particle arc trajectory satisfying dose heterogeneity on a 1D scanning machine, in accordance with an embodiment.
FIG. 14 illustrates optimization results with different healthy tissue sparing goals, in accordance with an embodiment.
FIGS. 15A-15B illustrates example dose distribution results without and with GRID condition optimization, in accordance with an embodiment.
FIG. 16A-16B illustrates example FLASH and GRID condition statistic results with different healthy tissue sparing goals, in accordance with an embodiment.
FIG. 17 illustrates a flowchart showing a technique for protecting healthy tissue in particle therapy, in accordance with an embodiment.
FIG. 18 illustrates generally an example of a system, such as may include a particle therapy system controller, in accordance with an embodiment.
FIG. 19 illustrates generally an example of a radiation therapy system, such as may include a particle treatment system and an imaging acquisition device, in accordance with an embodiment.

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.

### DETAILED DESCRIPTION

The systems and techniques described herein include an improvement to healthy tissue sparing in radiotherapy, including in scenarios involving delivery of a dose in ultra-high dose rates several orders of magnitude higher than conventional clinical radiotherapy. This type of dose delivery is known as FLASH radiotherapy. Many studies have shown that ultra-high dose rate may reduce normal tissue toxicities while maintaining local tumor control (this is known as the FLASH effect). A common approach to FLASH radiotherapy is to deliver a dose to a whole target in very short periods of time, which requires new hardware or hardware changes. Some previously proposed solutions lead to degraded treatment plan quality.

The systems and techniques described herein focus on delivering a dose to healthy tissue regions within a short period of time. Other techniques attempt to deliver a dose to the whole target in the short period of time. The current approaches focus on the period of time delivered to the healthy tissue regions rather than the entire target as a whole (e.g., longer delivery to the entire target may occur). These systems and techniques may be applied to any type of radiotherapy that provides sufficiently high dose rates and sufficient degrees of freedom allowing additional temporal optimization.

Another type of healthy tissue sparing in radiotherapy takes advantage of tissue sparing effects due to changing low and high dose regions in a close neighborhood, as compared to conventional and more homogenous dose distributions. A neighborhood may comprise immediate neighboring voxels or voxels within a short distance (e.g. several millimeters, several voxels, etc.). A neighborhood may further include a particular set of voxels or area within a search direction (e.g., searching in lateral spot direction only). This healthy tissue sparing using a neighborhood is known as GRID radiotherapy. A common approach to GRID radiotherapy is to utilize additional hardware (e.g., blocks with slits).

The systems and techniques described herein focus on creating dose heterogeneity via optimization. These systems and techniques may be applied to any type of radiotherapy that allows for reasonably fine spatial dose resolution and provides sufficient degrees of freedom allowing for additional spatial dose optimization. In some examples, both FLASH and GRID systems and techniques described herein may be combined to achieve maximum healthy tissue sparing, such as by optimizing for a suitable temporal-spatial dose distribution.

Particle arc therapy with spot scanning is particularly interesting for the present approaches due to a large degree of freedom in optimization. The systems and techniques described herein include the temporal or spatial dose heterogeneity properties of irradiation of healthy tissue regions into optimization. This optimization may be accomplished either by optimizing the spot trajectory or by spot weight optimization or by combination of both. An example benefit of this optimization is that it may use existing devices and hardware (for example, a particle spot scanning system). Another benefit includes maintaining desired treatment plan quality.

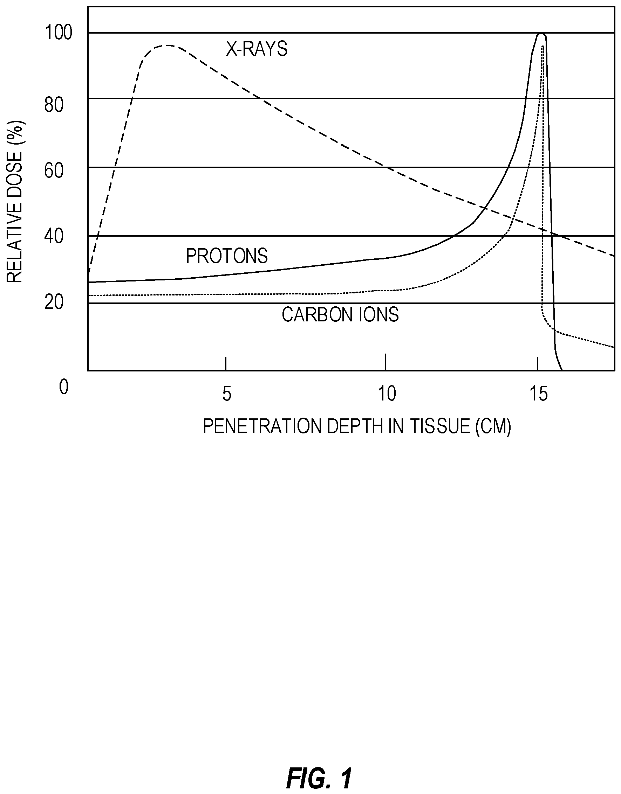

FIG. 1 provides an illustration of a comparison of radiation dose depths for various types of particles in human tissue. As shown, the relative depth of penetration into human tissue of photons (e.g., x-rays) versus protons versus carbon ions is provided (e.g., including any radiation dose provided at a distance beneath the surface, including secondary radiation or scatter). Each radiation dose is shown relative to the peak dose for a proton beam having a single energy which has been set to 100%.

The photon beam (e.g., labelled as X-rays) indicates the initial build up due to electron scatter. The mono-energetic (e.g., single energy) proton beam indicates a plateau region starting at approximately 25% that gradually increases until approximately 10 cm depth in tissue where it rapidly increases to the Bragg Peak at 15cm and then advantageously falls to zero within a short distance. No additional dose is delivered at the end of the Bragg peak. With normalization (e.g., scaling) at 15 cm depth, the dose due to x-rays is at 40% of the dose provided by proton beam, while the x-ray beam has a peak dose of greater than 95% at approximately 3cm depth.

Therefore, with x-rays, a considerable amount of dose is delivered prior to the target and an appreciable amount of dose is delivered past the target. The mono-energetic carbon beam shows a similar dose profile with mono-energetic proton beam but with lower entrance dose, sharper Bragg Peak and a tail (e.g., known as a "spallation tail", where some of the Carbon nuclei shatter into Helium ions) that has approximately 10% additional dose, or less, past the desired target by several centimeters. High energy particles (e.g., protons or carbon ions) has a significant reduction of dose to organ at risk (OAR). Further advantages include lower dose per treatment for the same biological effect to the tumor, which lowers the risk of side effects and may improve quality of life during and after particle therapy treatment.

One method of providing particle therapy is to use a broad beam, such as a spread-out Bragg peak that provides a uniform beam having multiple energies. If various energy fields are to be used to treat the patient, it may not be accomplished using a broad beam because too many compensators are required. Another issue with using a broad beam is there is an undesired shape to the dose at the proximal edge of the targeted tumor. Another method of providing particle therapy is Pencil Beam Scanning. Each particle beam comprises of one or more energies defining energy layers to control the depth at which particles deposit their energy and multiple thin particle spots within these energy layers defining the lateral beam extension. A particle spot is typically defined by a point in the beam coordinate system for a given energy layer. Particle spots are delivered using scanning magnets that define their lateral position. The spots within a beam scan a section of a target following a precise trajectory, thereby treating the tumor volume in a series of energy layers, spot positions and directions.

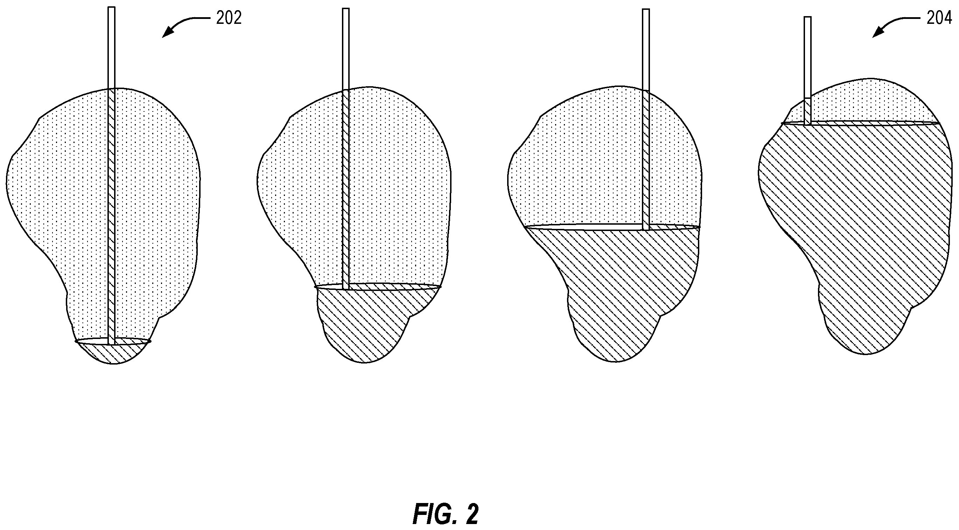

FIG. 2 provides an illustration of a Pencil Beam Scanning of an irregular shape volume from a distal edge (e.g., bottom) to a proximal (e.g., top) edge. As shown, the irregular shaped tumor volume is irradiated layers of protons. For example, a first time snapshot 202 shows a first layer of protons being delivered, and a later time snapshot 204 shows that most of the layers have been delivered. Each layer has its own cross-sectional area to which the protons having the same energy are delivered. The total radiation dose is provided as a layer-by-layer set of beamlets. Each layer of may have different energies. The most common means of specifying and delivering the set of beamlets to the cross-sectional area is to define and deliver beamlets having a constant diameter ("spot size") to a selection of grid points on each layer. While the majority of the dose from the beamlet is delivered to the targeted layer, a significant amount of dose is delivered along the path to the targeted layer. The dose to proximal layers from beamlets defined for distal layers is accounted for in the specification of the beamlets defined for the proximal layers. The ability to individually specify the number of particles (e.g., the meterset) for a given beamlet ensures that each part of the volume being irradiate receives the desired dose.

As shown in FIG. 2, a variety of different energy layers cover different cross-sectional areas of an irregular shaped tumor to which the particles having the same energy are delivered. A group of particles having the same energy determine the lateral size of the energy layer.

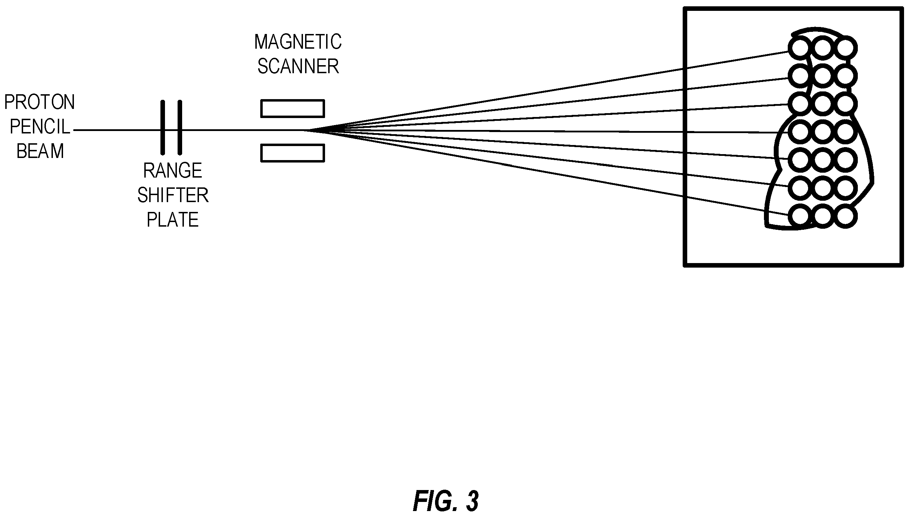

FIG. 3 provides an illustration of a diagrammatic representation of a typical active scanning proton beam delivery system. As shown, a single layer of a pencil beam scan is being delivered, with a grid of spots depicted on a patient in conjunction with a contour of the cross-sectional area to which particles are to be delivered. An incoming mono-energetic proton beamlet has a specified amount of its energy absorbed by the Range Shifter (e.g., in FIG. 3 it is a Range Shifter plate), resulting in a beamlet with the desired energy to achieve a certain depth for the Bragg Peak in the patient to treat the specified layer. The scanning proton beam delivery system includes a magnetic scanner, which has the ability to deflect the particles in both a vertical and a horizontal direction. The strength of the magnetic fields may be adjusted to control the deflection in the direction perpendicular to the magnetic field.

The amount of radiation passing through a spot is measured in MU or number of particles. Thus, if the total beam MU or number of particles is known, an equivalent quantity is the relative spot weight associated with each spot. The weight describes the fluence of radiation for a given beam energy. Each spot weight can be independently varied to create a modulated fluence.

The rate at which the magnetic field strengths may be adjusted determines the rate at which the scanning may take place. One common scheme for scanning is to scan in one direction quickly and to scan in the perpendicular direction more slowly in a raster fashion, similar to how early televisions were controlled (e.g., Cathode Ray Tube (CRT), which use electrons instead of protons), but arbitrary patterns may be scanned.

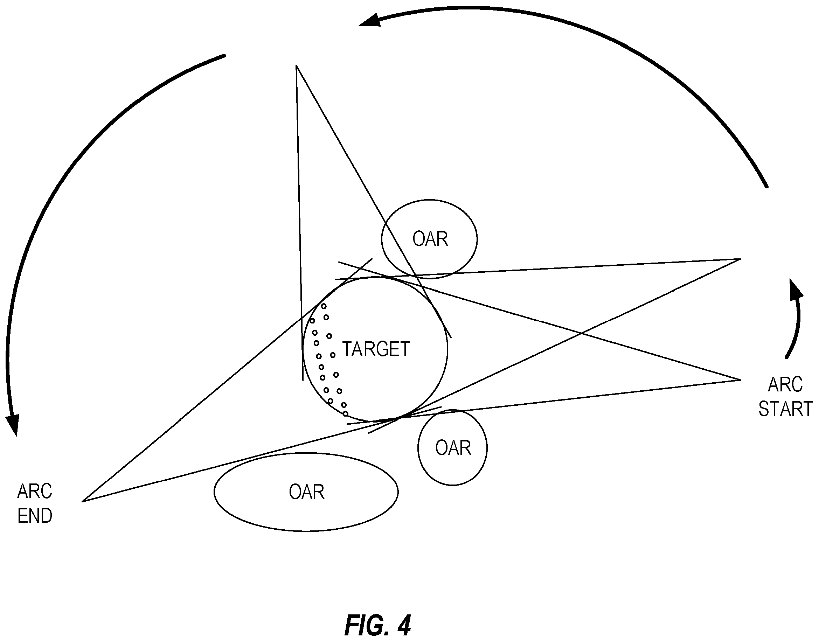

FIG. 4 illustrates a diagrammatic representation of a particle arc therapy, in accordance with an embodiment. A more advanced approach of delivering particle beam involves a rotating gantry during irradiation, which is called particle arc therapy. A particle arc beam may comprise a start and end angle and a series of nominal gantry angles in between, which may be evenly or unevenly distributed. At each gantry angle, one or more energy layers may exist covering a section of target and each energy layer contains one or more particle spots to cover different positions of target at the same depth laterally. Overall, a particle arc beam is composed of a plurality of beamlets from different gantry angles, energy layers and spot positions. Given the large degrees of freedom, a particle arc plan may be configured to achieve superior quality (e.g., improved conformality, improved healthy tissue sparing, etc.), such as compared to more conventional techniques. Moreover, particle arc therapy may also help improving delivery efficiency because it alleviates the burden on setting up and delivering multiple static beams while maintaining the same or even better plan quality.

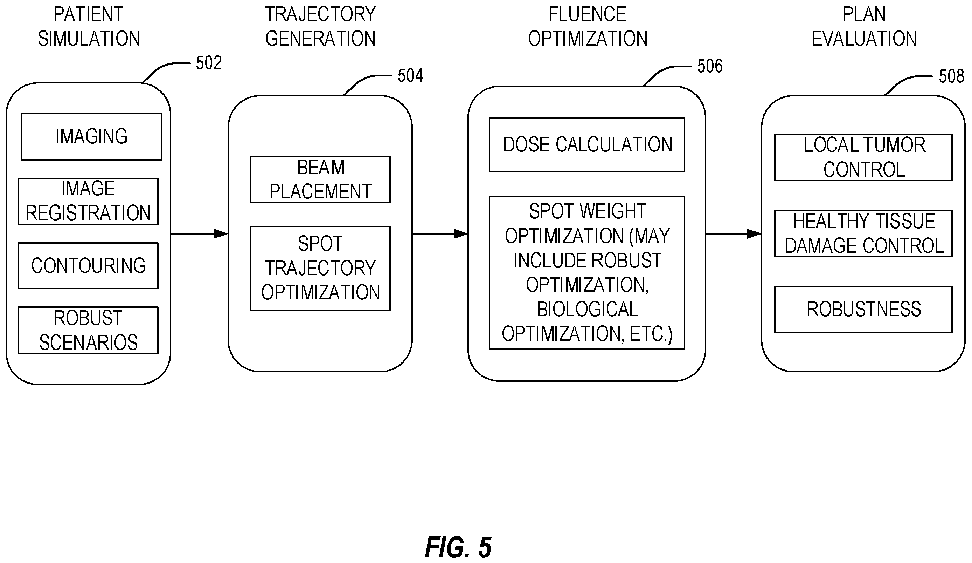

FIG. 5 illustrates a particle treatment planning system, in accordance with an embodiment. Treatment planning is a crucial step in radiation therapy. Given patient simulation information, treatment prescription, and machine properties, treatment planning provides a treatment plan including a series of varying delivery parameters. The parameters may be used by a machine to perform irradiation to fulfill the desired dose distribution. This procedure may be handled manually in some examples. In other examples, to improve adherence to the plan, to improve patient outcomes, and to delivery results more quickly, a more efficient technique may use a treatment planning system (TPS) to optimize the treatment plan automatically. This objective and automatic technique differs from human trial and errors, which require an immense amount of time and are subject to errors, such as based on subjective judgment. In terms of particle arc therapy, due to the large degrees of freedom for treatment planning, an automatic and efficient TPS outputs superior results. In U.S. Patent Application 17/302,225, a novel and efficient particle arc treatment planning solution was described. This planning solution may generate an efficient particle arc plan automatically.

FIG. 5 provides a schematic flowchart of an example particle treatment planning system. In a patient simulation step 502, patient image datasets (e.g., optionally from variety of imaging modalities) are acquired. This step 502 may include imaging at a different time than the planning or procedure (e.g., before) or may be acquired at a planning time. During step 502, image registration may occur, such as between different imaging modalities or different imaging phases. Image registration may include additional patient simulation, for example performed on multiple robust scenarios. Step 502 may include contouring or identifying treatment targets and OARs. After step 502, a trajectory generation step 504 may occur. In step 504, one or more beams with multiple spots in a precise order (e.g., called a trajectory) may be optimized. Step 504 may include identifying beam placement and optimizing spot trajectory. An optimized trajectory may include one that satisfies a number of treatment plan requirements, such as sufficient target coverage, OAR sparing, acceptable delivery efficiency, etc.

After the trajectory is optimized, a fluence optimization step 506 may occur. The fluence optimization step 506 may include a dose calculation and spot weight optimization to achieve a desired plan quality in dose aspects. This may include robust optimization, biological optimization, or the like. After fluence optimization, a plan evaluation step 508 may occur. In step 508, tools for local tumor control or heathy tissue damage control may be evaluated. In some examples, robustness evaluation may occur during step 508.

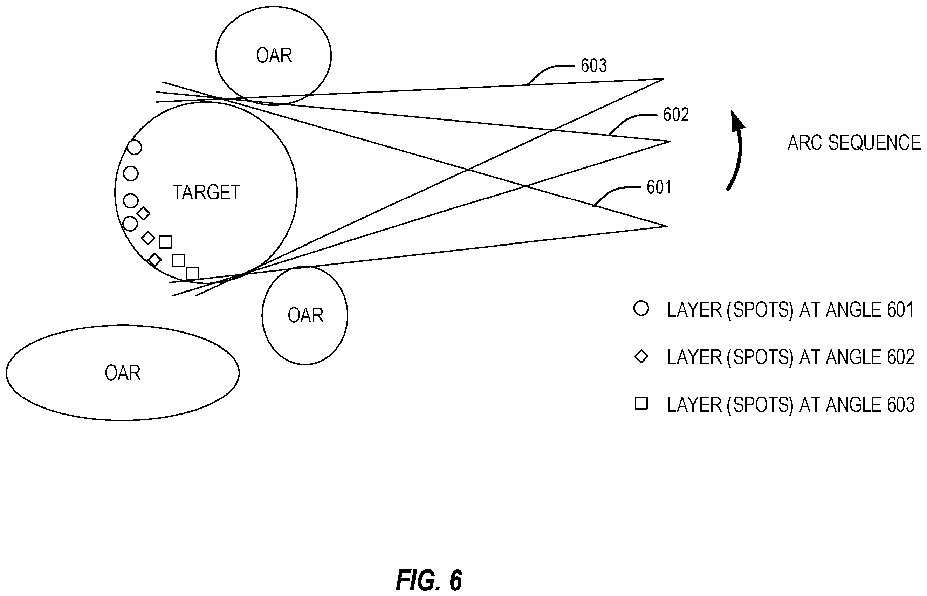

FIG. 6 illustrates an example particle arc trajectory, in accordance with an embodiment. FIG. 6 includes three sequential gantry angles 601, 602, and 603. In this example, gantry 601 uses a highest energy to reach a distal end of the target. Several spots (e.g., shown as circular points in FIG. 6) are placed at this energy layer and cover the distal part of the target. Gantry 602 then irradiates a new layer (e.g., slightly shallower, shown as diamond points in FIG. 6) from a nearby angle while skipping the area that has been irradiated by a nondifferentiable angle via gantry 601. The nondifferentiable angle includes the angular difference that is not sufficient to create a differentiable dose distribution. Gantry 603 irradiates another new layer (e.g., shown as rectangular points in FIG. 6) and skips the already covered areas from gantry 601 and 602. The order of spots within a single layer may be optimized to achieve a faster layer scanning time or better delivery quality (e.g., the spot trajectory may be optimized in a layer that more off-central axis spots are delivered around a nominal angle to minimize the predicted and delivered dose error).

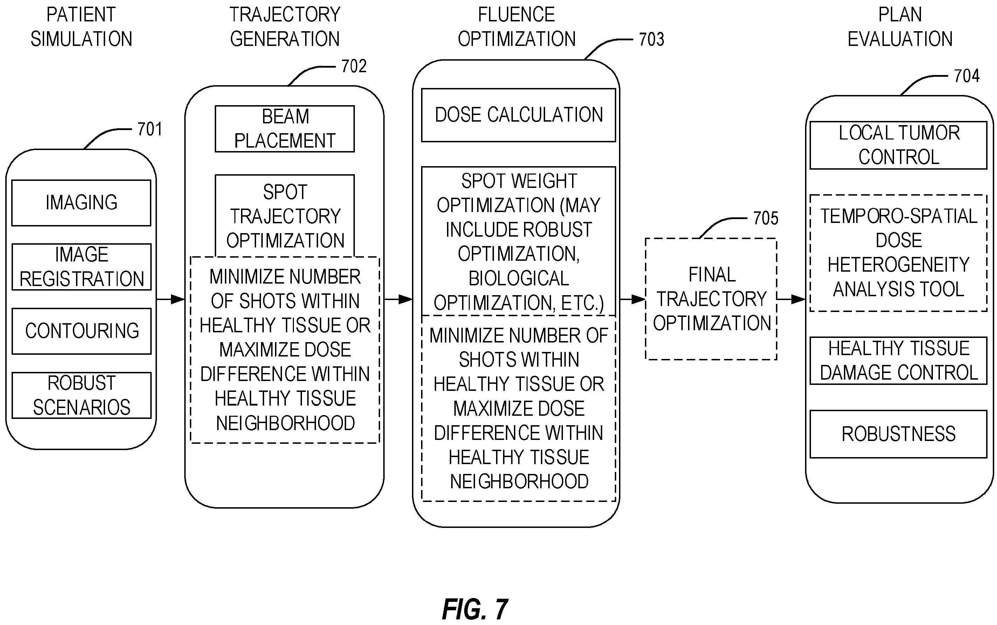

FIG. 7 illustrates an extended particle treatment planning workflow for optimizing dose heterogeneity effects, in accordance with an embodiment. FIG. 7 includes some elements similar to those discussed above with respect to FIG. 5 (e.g., step 703, which may include similar imaging, image registration, contouring, or robust scenario evaluation as discussed in step 502 of FIG. 5), and also introduces additional examples and workflow steps (e.g., optional step 705 for final trajectory optimization between steps 703 and 704). FIG. 7 includes an optional modification to trajectory generation in step 702 related to minimizing a number of shots within healthy tissue, or maximizing a dose difference within a healthy tissue neighborhood. FIG. 7 includes an optional modification to fluence optimization in step 703 related to minimizing a number of shots within healthy tissue or maximizing a dose difference within a healthy tissue neighborhood. FIG. 7 includes an optional modification to plan evaluation in step 704 related to temporo-spatial dose heterogeneity, such as using an analysis tool. These steps are discussed in further detail below.

Particle spots and single energy layers may be delivered very quickly with existing delivery methods. However, in order to cover the target with sufficient dose, a contribution of multiple energy layers from one or more gantry or couch angles is needed. This typically leads to non-FLASH conditions on healthy tissue as the healthy tissue receives dose contribution from multiple energy layers. In order to minimize the non-FLASH conditions to healthy tissue due to overlap of dose contribution from different energy layers and angles, the systems and methods disclosed herein optimize a treatment plan with this additional goal.

When healthy tissue needs to receive a higher dose (e.g., typically because a spot traverses it on the way to or from the target), the number of times such healthy tissue gets hit by higher dose may be restricted. This may happen in two ways (or combination of both) in the treatment planning workflow.

One approach to spare healthy tissue temporally includes spot trajectory optimization, such as with an additional objective of minimizing high dose overlap of spots from different energy layers or spot groups (e.g., step 702 in FIG. 7). This step 702 may include having each healthy tissue voxel receive a dose in a minimum number of shots. In one implementation, high dose overlap may be measured by number of paints on healthy tissue. Painting a voxel may include, for example, that the voxel stays within the high dose region of a spot. The painting number may be quantified in different ways.

Another optimization approach includes fluence optimization. This is discussed in more detail below.

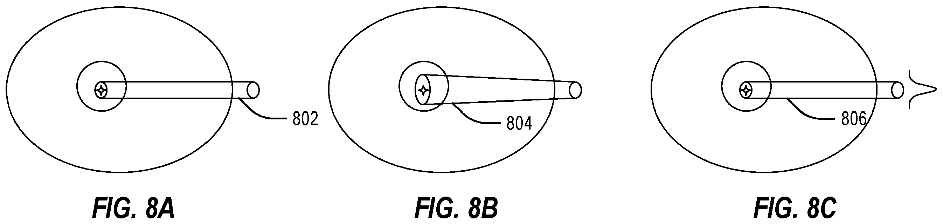

FIGS. 8A-8C illustrate examples of a high dose region, in accordance with an embodiment. FIG. 8A illustrates an example of a cylindrical high dose region 802 around a central axis of a particle beamlet. The lateral diameter of the high dose region 802 may be defined by a fixed and approximate spot size. Every voxel within the cylinder may be counted as one paint. FIG. 8B illustrates another example of a cone shape high dose region 804, which also centers around the beamlet axis but has an increasing lateral diameter along the beamlet direction. The increasing lateral diameter may be estimated by the depth and a scattering mode. Every voxel within the cone 804 may be counted as one paint. FIG. 8C illustrates yet another example of cylindrical high dose region 806 with weighted (for example using a gaussian function) painting number within the region 806. The closer to the central axis, the higher painting number a voxel is assigned. In an example, the number of paints of healthy tissue may be limited to zero or one using this type of region 806 with weighting. In this example, every healthy tissue voxel may receive its total dose all in one shot (or not at all), which matches a FLASH-like condition. In other examples, minimizing the number of paints of healthy tissue may be improved with this region 806 with weighting, such as to help achieve a sufficient part of healthy tissue fulfilling a FLASH-like condition.

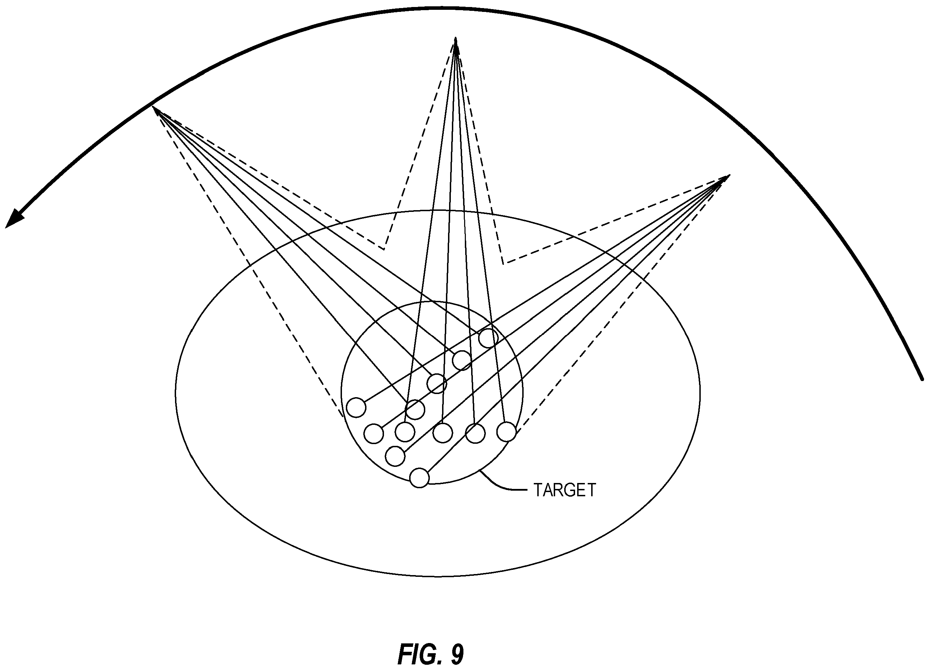

FIG. 9 illustrates an example particle arc trajectory satisfying dose heterogeneity by avoiding high dose region overlapped outside the target, in accordance with an embodiment.

In the example particle arc beam shown in FIG. 9, each particle gantry angle includes one energy layer with multiple spots. In this example, the spot positions and layer energies may be selected such that no healthy tissue is painted more than once by a spot group delivered within a short period of time. In this example, the target still receives sufficient high dose coverage. As a result of such well optimized spot trajectory, healthy tissue may be protected due to well satisfied FLASH-like conditions.

Another example approach to fulfill FLASH-like conditions includes spot weight optimization with additional objective, or constraint, or penalizing function to reduce the number of shots on healthy tissue (e.g., step 703 as discussed with respect to FIG. 7). In this example, one shot may indicate that irradiation is accomplished within a sufficient short period of time which satisfies FLASH-like conditions. A total dose on healthy tissue may be delivered by one or more shots (e.g., a minimized number of shots), and with the smaller number of shots on healthy tissue, a better FLASH-like condition is met. In an example, every healthy tissue voxel may receive a total dose delivered in one shot. In an example, the FLASH-like condition may be achieved by introducing a penalizing function (which can be also used as a constraint or additional objective), such as described in Eq. 1: $f = \frac{1}{N} ∗ {\sum }_{i = 1}^{N} \frac{{\left({\sum }_{j = 1}^{M} {ω}_{j} {d}_{ij}\right)}^{2}}{{\sum }_{j = 1}^{M} {\left({ω}_{j}{d}_{ij}\right)}^{2}}$

In Eq. 1, "i" specifies the healthy tissue region (for example a voxel), N is the total number of healthy tissue regions to consider (this non-targeted tissue to consider can be for example specific user selected structures, the whole tissue outside the target etc.), j represents a particle spot group, M represents a total number of spot groups, ω_j is a weight of spot group j, and d_(i,j) is a dose to healthy tissue region i contribution from spot group j. A spot group may include a single spot, spots delivered within predefined short period of time, all spot within a layer, etc.

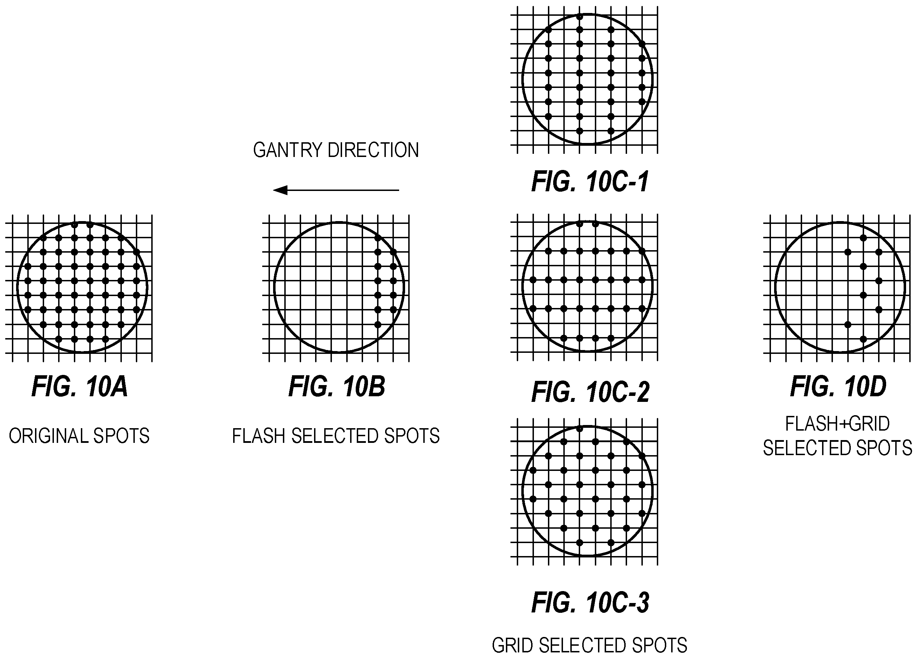

FIGS. 10A-10D illustrate examples of different spot trajectories on a single energy layer, in accordance with an embodiment.

As a result of spot trajectory optimization or fluence optimization with the additional goal or constraint of generating temporal dose heterogeneity, a given spot trajectory per layer (e.g., containing only valid spots which have fluence greater than zero) may include a long island shape. FIGS. 10A-10B show some examples of different spot trajectory results of a single layer under different circumstances. FIG. 10A illustrates a classic single layer trajectory, which fully covers the tumor cross-section at a certain depth. In FIG. 10B, spots in one layer cover only a section of the tumor at this depth. In this example, the selected spots form a long island shape of which the long axis is perpendicular to the gantry rotation direction. This pattern may be used to avoid high dose overlap between layers from consequential angles.

After spot weight optimization, an optional final spot trajectory optimization step may be applied (e.g., step 705 as discussed with respect to FIG. 7). In order to fulfill a FLASH effect, considerable spot weights may be optimized to zero to avoid undesired dose in healthy tissues. For example, final spot trajectory may include generating several "spot islands" or "holes" within a layer. Performing a final spot trajectory optimization may be helpful to deliver spots faster in layers.

As a result of spot trajectory optimization or fluence optimization with the additional goal or constraint of generating spatial dose heterogeneity, a given spot trajectory per layer (e.g., containing only valid spots which have fluence greater than zero) may include multiple spot islands. As discussed above with respect to a classic single layer trajectory in FIG. 10A, which fully covered the tumor cross-section at a certain depth, FIGS. 10C-1, 10C-2 10C-3 are three different examples of valid spots in one layer modified by spatial dose heterogeneity optimization. Each of these examples results in spots that form an interleaved shape in horizontal direction (FIG. 10C-1), vertical direction (FIG. 10C-2), or 2D directions (FIG. 10C-3). This interleaving may be used to create spatial dose heterogeneity.

As a result of a combination of both temporal and spatial dose heterogeneity optimization, a final spot trajectory in one layer may include a long island shape with holes, such as the results shown in FIG. 10D. The result shown in FIG. 10D is an effect of sparing overlapped doses from layers to layers (e.g., spot groups to spot groups) and from spots to spots within one layer (e.g., in one spot group).

A final spot trajectory optimization step (e.g., step 705 as discussed with respect to FIG. 7) may be used for spatial dose heterogeneity optimization. The final optimization may include removing several or considerable spots, such as to avoid undesired dose in healthy tissue neighborhood. This may result in several "spot islands" or "holes" within one layer. The final spot trajectory optimization may be helpful to deliver spots faster in layers.

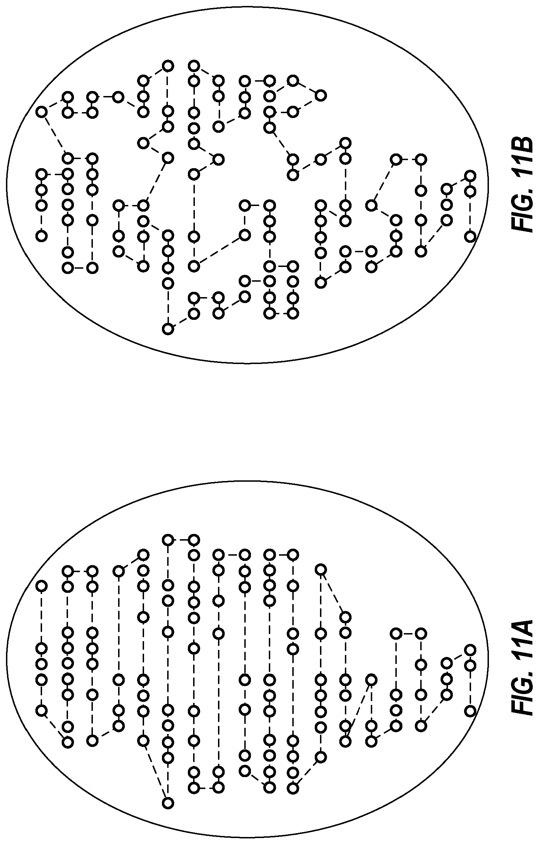

FIGS. 11A-11B illustrate an example of a spot trajectory with 'holes' on one layer after weight optimization, in accordance with an embodiment. FIG. 11A includes an example of one layer with valid spots after weight optimization, and several "holes" are visible (e.g., spaces dashed between spots). FIG. 11B illustrates a different spot trajectory with the same spots as in FIG. 11A. In an example, the spot trajectory of FIG. 11B is faster in delivery than that shown in FIG. 11A.

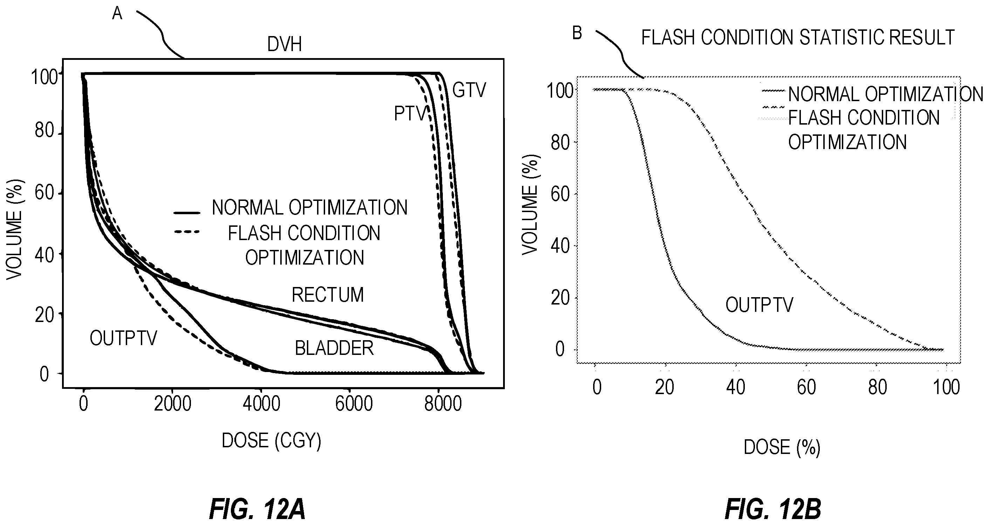

FIGS. 12A-12B illustrate example FLASH condition optimization results, in accordance with an embodiment. In a plan evaluation step (e.g., step 704 as discussed with respect to FIG. 7), a new model may be used, specifically for FLASH-condition review. One example in FIG. 12A shows a prostate proton arc plan using the penalizing function in Eq. (1). The penalizing function effects on "outPTV" structure, which is a surrounding structure of PTV and all the rest of healthy tissues are beyond it. When "outPTV" satisfies FLASH-like conditions then the whole patient volume is well protected. Results in FIG. 12A show well maintained plan quality in dose distribution. In an example, a FLASH condition statistic result in FIG. 12B illustrates a better FLASH-like condition, achieved by FLASH condition optimization, than in FIG. 12A. The FLASH condition statistic result of FIG. 12B includes a cumulative volume histogram of percentage dose delivered under FLASH-like conditions (e.g., the dose is delivered within a single layer scanning time, which may be typically less than a second, less than half a second, etc.).

The extended workflow of FIG. 7 illustrates the possibility of healthy tissue protection by optimizing dose heterogeneity spatially to achieve GRID-like conditions. For example, in step 702 as discussed with respect to FIG. 7 spot trajectory optimization is performed with an additional objective of maximizing the dose difference within the healthy tissue neighborhood. Similarly, dose difference may be measured by number of paints by different spot groups with sufficient spatial distance of their high dose regions within healthy tissue neighborhood. By minimizing the number of paints within the healthy tissue neighborhood, a sufficient spatial dose heterogeneity may be achieved. The healthy tissue may be better protected by minimizing the number of paints.

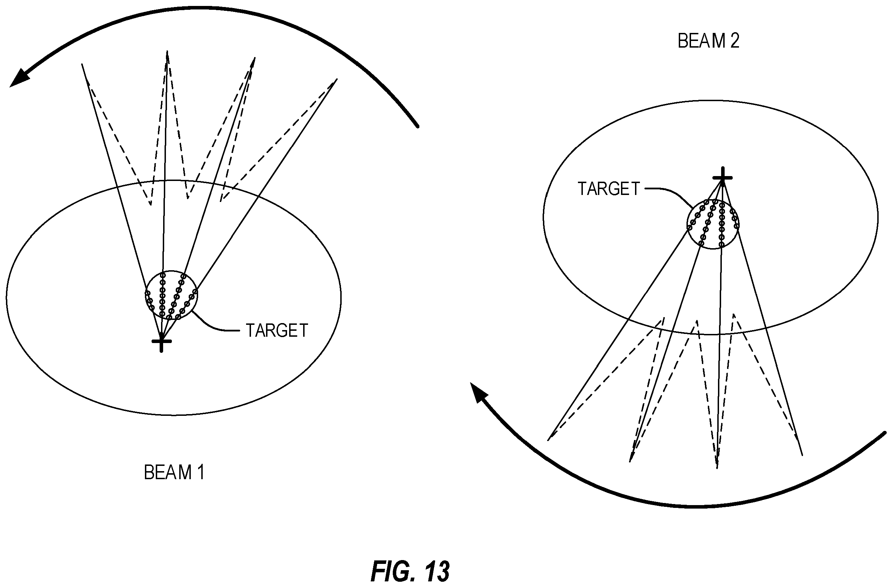

FIG. 13 illustrates an example particle arc trajectory satisfying dose heterogeneity on a 1D scanning machine, in accordance with an embodiment.

In an example, a particle arc beam may scan along one axis only. By adjusting energy and scanning magnet field intensity, one particle gantry angle may irradiate a thin plane (e.g., the beam central axis - scanning direction plane) of the target. Beam 1 in FIG. 13 rotates from northeast to northwest in a counter-clockwise direction and the iso-center locates below the target. Each angle in Beam 1 scans a target slice. Due to beam setup geometry, there may be denser spots in a distal part of the target and sparser spots in a proximal part of the target. The healthy tissues that stay shallower than the target are well protected because they avoid undesired irradiation according to the sparse grid.

Beam 2 is setup opposite to Beam 1, which is introduced to mitigate a too sparse grid in the proximal region or too dense grid in the distal region of the target. A combination of Beam 1 and Beam 2 may be used to distribute a uniform target dose while preserving healthy tissues in GRID-like conditions. When energy switching is fast enough, FLASH conditions may be achieved by this approach as well, such as for a spot group delivered from same nominal angle. The number of beams selected in FIG. 13 is an example, the approach works for any number of beams. In some examples, the iso-center may be located within the target. In some examples, 2D scanning may be applied.

While FLASH trajectory optimization may result in single or zero paints of a spot or spot group delivered within a short time period to healthy tissue regions, the GRID trajectory optimization aims at finding spots such that sufficient lateral distance exists between spots or spot groups at given nominal positions leading to dose heterogeneity. In an example, both FLASH- and GRID-like trajectory optimization methods may be combined for achieving both temporal and spatial dose heterogeneity.

An approach to maximize spatial dose heterogeneity may include spot weight optimization with an additional objective, constraint, or penalizing function to increase the dose difference within the healthy tissue neighborhood (e.g., step 703 as discussed with respect to FIG. 7). In an example, the spatial dose heterogeneity may be achieved by introducing a penalizing function (which can be also used as a constraint or additional objective), such as described in Eqs. 2-3: ${d}_{i} = {\sum }_{j = 1}^{M} {ω}_{j} {d}_{ij}$ $f = \frac{1}{N} ∗ {\sum }_{i = 1}^{N} \frac{{{d}_{i}}^{2}}{\frac{1}{K - 1} ∗ {\sum }_{i ′ ∈ N br , i ′ \neq i} {\left({d}_{i}-{d}_{i ′}\right)}^{2}}$

In Eqs. 2-3, "i" specifies the healthy tissue region (for example a voxel), N is the total number of healthy tissue regions to consider (this non-targeted tissue to consider can be for example specific user selected structures, the whole tissue outside the target etc.), j represents a particle spot, M total number of spot, ω_j is weight of spot j, d_(i,j) is dose to healthy tissue region i contribution from spot j. di is the dose to healthy tissue region i, and Nbr is the neighborhood of the healthy tissue region i. K is the total healthy tissue region within the neighborhood Nbr. Eq. 3 may be used to increase the dose difference within each healthy tissue region neighborhood.

The additional objective, constraint, or penalizing function to achieve spatial dose heterogeneity may be combined with the additional objective, constraint, or penalizing function to achieve temporal dose heterogeneity such that temporo-spatial dose heterogeneity for healthy tissue regions may be achieved simultaneously.

Within the workflow described with respect to FIG. 7, for example, a combinations of all options may be used. For example, trajectory optimization may be performed in a standard way or for additional temporal heterogeneity or for additional spatial heterogeneity or for additional temporospatial heterogeneity. Fluence (weight) optimization may be performed in a standard way or for additional temporal heterogeneity or for additional spatial heterogeneity or for additional temporospatial heterogeneity.

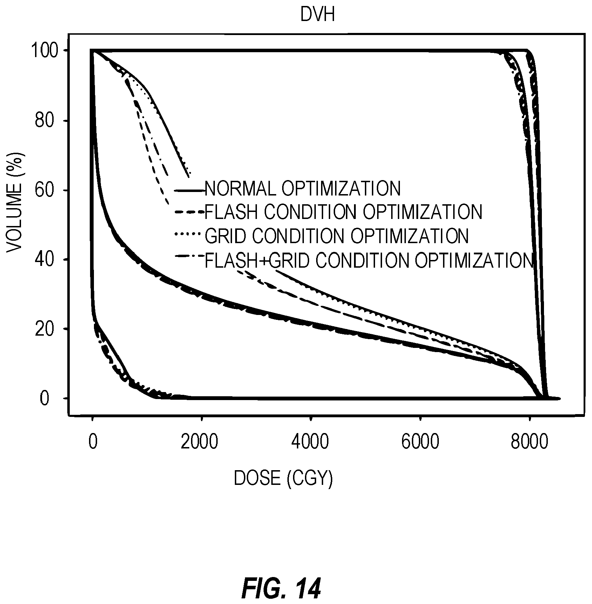

FIG. 14 illustrates optimization results with different healthy tissue sparing goals, in accordance with an embodiment. For example, FIG. 14 includes an example optimization dose-volume histogram (DVH) result on a prostate proton arc plan. The plan is optimized on different healthy tissue sparing conditions. In this example, a reference plan without any additional healthy tissue protection option is shown as a solid line, which achieves the best target coverage. Three other plans are shown which adopt temporal heterogeneity spot weight optimization, spatial heterogeneity spot weight optimization, and temporospatial heterogeneity spot weight optimization, respectively. In comparison to the reference plan, the other three plans have a target coverage that is slightly deteriorated by the additional healthy tissue sparing, but not by much. The quality of the other three plans in terms of tumor control are well maintained generally.

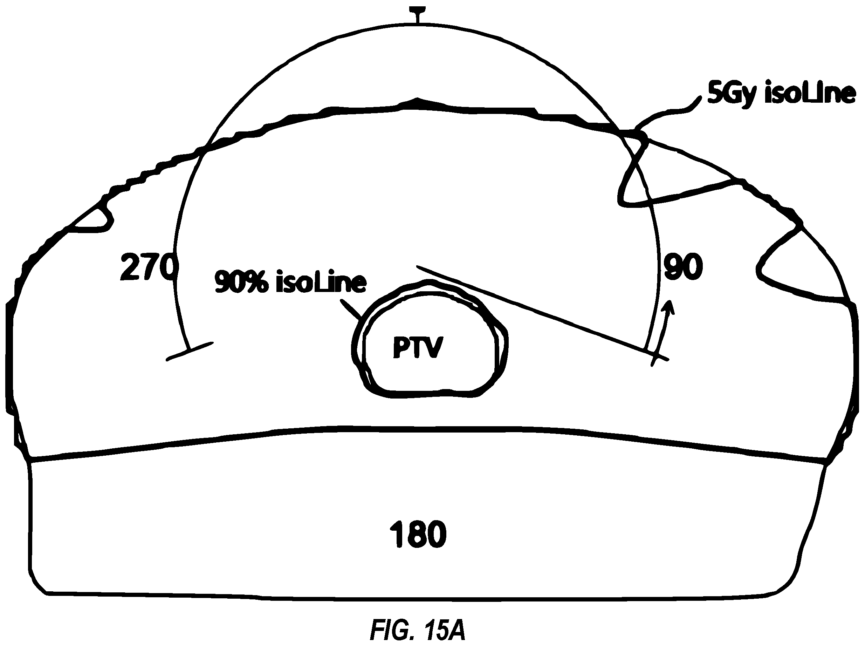

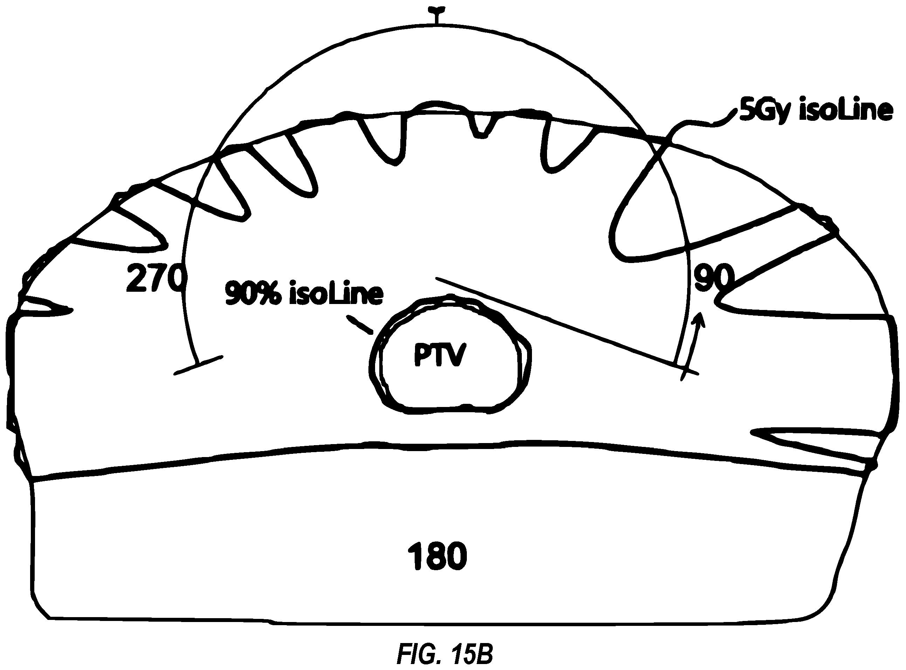

FIGS. 15A-15B illustrates example dose distribution result without (image 15A) and with (image 15B) GRID condition optimization, in accordance with an embodiment. FIG. 15A provides one example slice of 3D dose distribution without and FIG. 15B provides one example slice of 3D dose distribution with spatial heterogeneity penalty applied. The dose map on the bottom is more heterogeneous than the dose map on the top, illustrating the effect of the spatial heterogeneity penalty applied in the bottom image, but not the top.

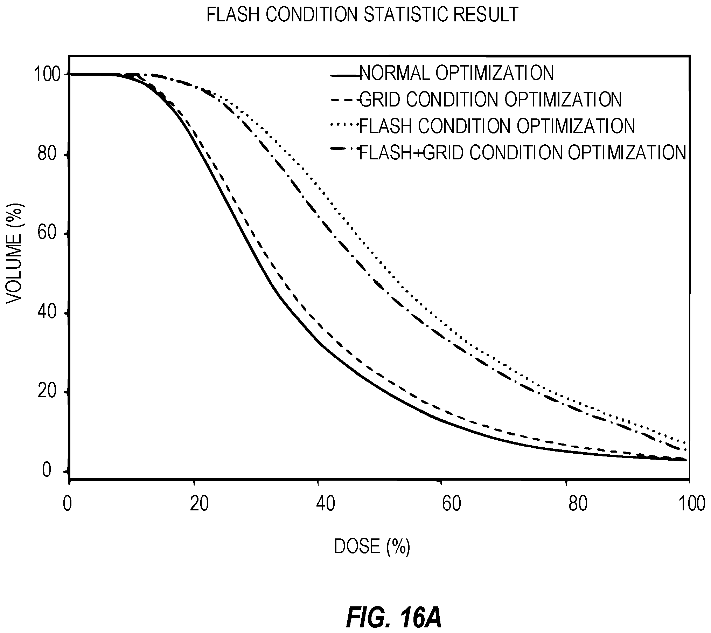

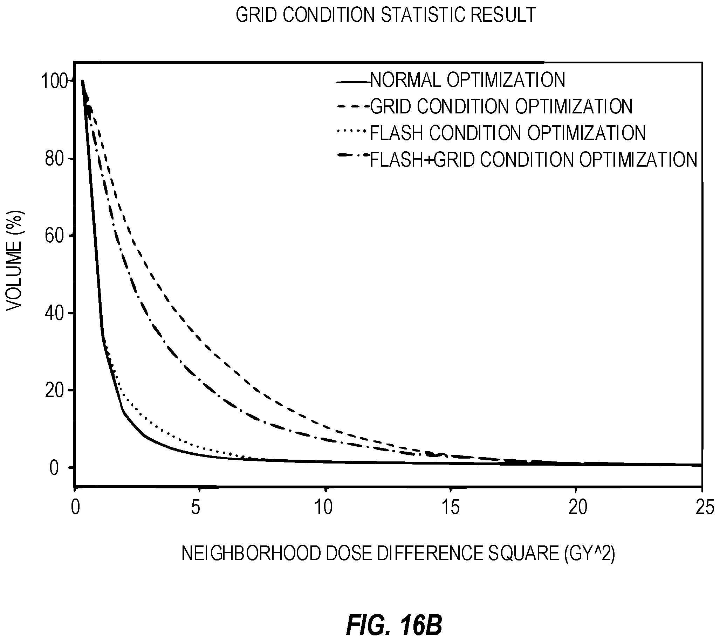

FIGS. 16A-16B illustrate example FLASH and GRID condition statistic results with different healthy tissue sparing goals, in accordance with an embodiment. FIG. 16A illustrates a FLASH condition statistic result and FIG. 16B illustrates a GRID condition statistic result.

FIGS. 16A-16B include an example of FLASH and GRID condition statistic results from the same plans optimized in FIG. 14. While preserving a satisfactory tumor control (e.g., as shown in FIG. 14), temporal or spatial heterogeneity optimization may achieve better healthy tissue sparing in different (e.g., temporal, spatial, or temporospatial) considerations. The FLASH condition statistic result of FIG. 16A shows cumulative volume histogram of percentage dose delivered under FLASH-like condition (in this case, for example, the dose is delivered within a single layer scanning time, such as within half a second or a second). The GRID condition statistic result of FIG. 16B shows cumulative volume histogram of average dose difference square (dose variance) in a neighborhood. The higher dose variance, the better GRID-like condition is met. Another option may include a cumulative volume histogram of dose standard deviation in a neighborhood. The comparison among different optimization options illustrates that the dose heterogeneity optimization goal helps with improving healthy tissue sparing.

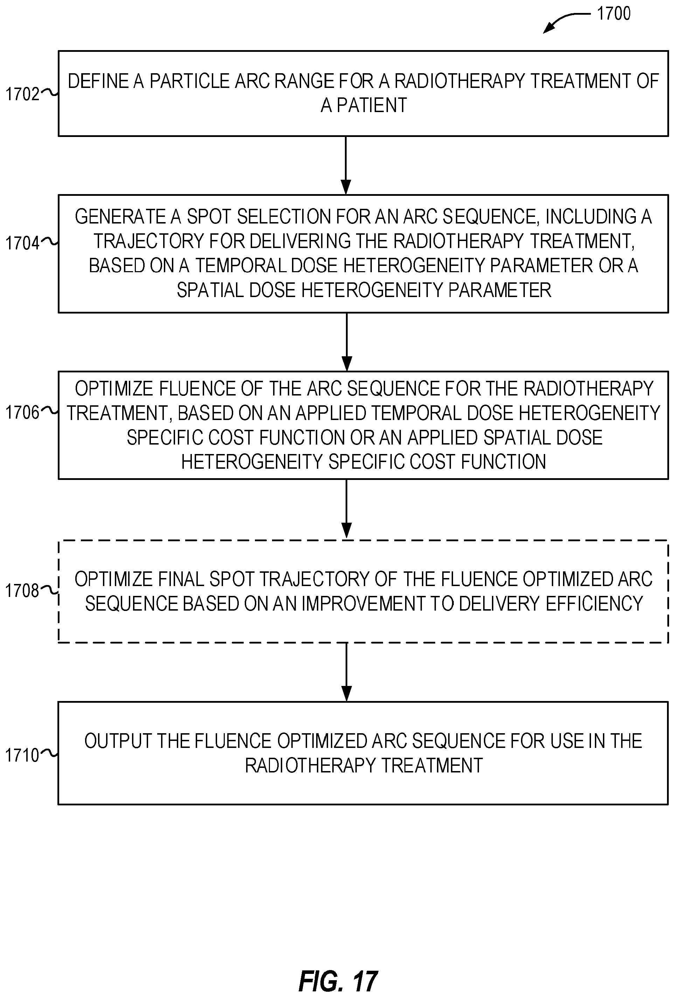

FIG. 17 illustrates a flowchart showing a technique 1700 for protecting healthy tissue in particle therapy, in accordance with an embodiment. The technique 1700 includes an operation 1702 to define a particle arc range for a radiotherapy treatment of a patient.

The technique 1700 includes an operation 1704 to generate a spot selection for an arc sequence, including a trajectory for delivering the radiotherapy treatment, based on a temporal dose heterogeneity parameter or a spatial dose heterogeneity parameter. Operation 1704 may include generating the spot selection based on both the temporal dose heterogeneity parameter and the spatial dose heterogeneity parameter.

When operation 1704 includes using the temporal dose heterogeneity parameter, selection of the temporal dose heterogeneity parameter may minimize a number of shots within healthy tissue. In this example, the number of shots represent a number of paints from different scanning layers or spot groups delivered within a specified time period on the healthy tissue. The number of paints may be minimized, such as to zero or one in some examples. The spot groups may include single spots, multiple spots within a single energy layer, single spots within multiple energy layers, multiple spots within multiple energy layers, or the like. The number of shots may represent a high dose overlap from different scanning layers or spot groups delivered within a specified time period on the healthy tissue. In this example, minimizing the number of shots may include using a penalizing function (which can be also used as a constraint or additional objective). An example penalizing function may include Eq. 1, discussed above. Selection of the temporal dose heterogeneity parameter may minimize a number of shots within non-targeted tissue. In an example, non-targeted tissue may include healthy tissue, an OAR, a subsection (e.g., a user-created contour or a specific shape), a sub-volume (e.g., a voxel), a combination of any of these, or the like. A number of shots delivered to the non-targeted tissue may include reducing a number of shots within a sub-target within non-targeted tissue, in an example.

When operation 1704 includes using the spatial dose heterogeneity parameter, selection of the spatial dose heterogeneity parameter may be used to maximize a dose difference within a healthy tissue neighborhood. In this example, the maximized dose difference may be measured in number of paints by different spot groups having a minimum spatial distance of high dose regions within the healthy tissue neighborhood. The number of paints may be minimized, such as by being limited to zero or one. Maximizing the dose difference in some examples may include using a penalizing function (which can be also used as a constraint or additional objective), such as Eqs. 2 and 3, as discussed above.

The technique 1700 includes an operation 1706 to optimize fluence of the arc sequence for the radiotherapy treatment, based on an applied temporal dose heterogeneity specific cost function or an applied spatial dose heterogeneity specific cost function. Operation 1706 may include optimizing the fluence based on both the applied temporal dose heterogeneity specific cost function and the applied spatial dose heterogeneity specific cost function.

When operation 1706 includes using the applied spatial dose heterogeneity specific cost function, operation 1706 may include minimizing a number of shots within healthy tissue. In this example, the number of shots represent a number of paints from different scanning layers or spot groups delivered within a specified time period on the healthy tissue. The number of paints may be minimized, such as by being limited to zero or one. The spot groups may include single spots, multiple spots within a single energy layer, single spots within multiple energy layers, or multiple spots within multiple energy layers. In an example, the number of shots represent a high dose overlap from different scanning layers or spot groups delivered within a specified time period on the healthy tissue. In this example, minimizing the number of shots may include using a penalizing function (which can be also used as a constraint or additional objective), such as Eq. 1, as discussed above.

When operation 1706 includes using the applied temporal dose heterogeneity specific cost function, operation 1706 may include maximizing a dose difference within a healthy tissue neighborhood. In an example, the maximized dose difference is measured in number of paints by different spot groups having a minimum spatial distance of high dose regions within the healthy tissue neighborhood. In this example, the number of paints may be minimized, such as by being limited to zero or one. Maximizing the dose difference in some examples may include using a penalizing function (which can be also used as a constraint or additional objective), such as Eqs. 2 and 3, as discussed above.

The technique 1700 includes an optional operation 1708 to optimize final spot trajectory of the fluence optimized arc sequence based on an improvement to delivery efficiency. The technique 1700 includes an operation 1710 to output the fluence optimized arc sequence for use in the radiotherapy treatment. Operation 1710 may include outputting the fluence optimized arc sequence for display. Operation 1710 may include outputting the fluence optimized arc sequence to cause the radiotherapy treatment to occur. Operation 1710 may include saving the fluence optimized arc sequence for use in planning the radiotherapy treatment. In an example, the technique 1700 may include displaying, for example on a user interface, a visualization of a portion of the arc sequence including a trajectory and delivery parameters on a medical image or a model of anatomy of the patient. In an example, the fluence optimized arc sequence is used in particle delivery via intensity modulated proton therapy (IMPT), a proton arc, a proton arc with 1D-only lateral scanning, or the like.

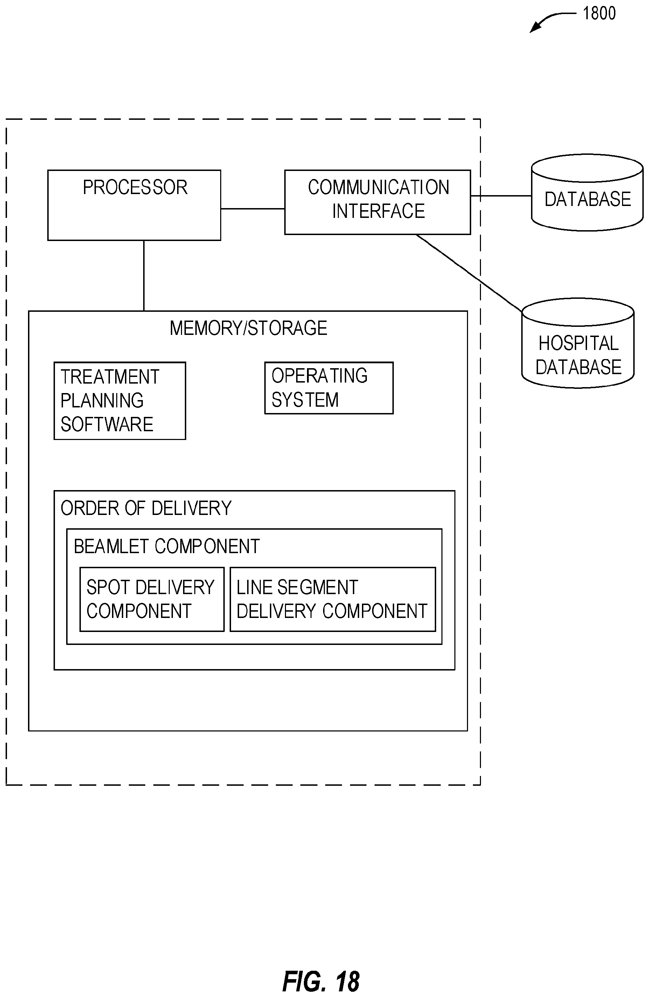

FIG. 18 illustrates generally an example of a system 1800, such as may include a particle therapy system controller, in accordance with an embodiment. The system 1800 may include a database or a hospital database. The particle therapy system controller may include a processor, communication interface, or memory. The memory may include treatment planning software, an operating system, or a delivery controller. The delivery controller may include a beamlet component for determining or planning spot delivery (e.g., using a spot delivery component) or line segment delivery (e.g., using a line segment delivery component).

In an example, the spot delivery component or the beamlet component may be configured to plan size of beamlets, location of a target or spot, or the like. The beamlet component may be used to determine an order of delivery of beamlets, for example in a spiral pattern as described herein. The order of delivery component may be in communication with the treatment planning software for planning delivery of beamlets. For example, the treatment planning software may be used to determine or plan gantry angle, gantry speed, beamlet size, spiral pattern (e.g., clockwise or counterclockwise), angle range for a particular spiral pattern (e.g., every ten degrees of the gantry rotation), or the like.

The processor may implement the plan, such as by communicating, via the communication interface or otherwise, to components used to implement the plan (e.g., to control devices or components). In an example, the communication interface may be used to retrieve stored information from a database or a hospital database (e.g., patient information, past procedure information for the patient or other patients, procedure instructions, information about particular devices or components, or the like).

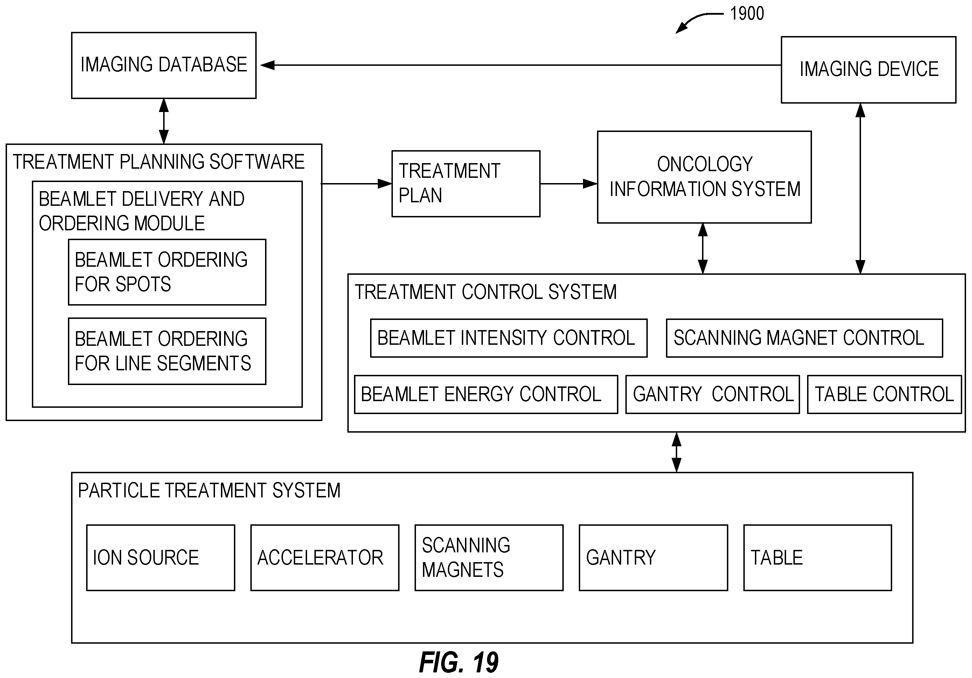

FIG. 19 illustrates generally an example of a radiation therapy system 1900, such as may include a particle treatment system and an imaging acquisition device, in accordance with an embodiment. The particle treatment system includes an ion source, an accelerator, and scanning magnets. The particle treatment system includes a gantry and a table, where the gantry may be mounted on the table, affixed to the table, or stabilized with respect to the table. The table may hold a patient. The gantry may be a rotating gantry, and may rotate with respect to the table (e.g., around the table) or with respect to the patient (and the table or a portion of the table may rotate with the gantry).

The particle treatment system may communicate with a treatment control system, which may be used to control actions of the particle treatment system. The treatment control system may communicate with an imaging acquisition device (e.g., to receive images taken by the imaging acquisition device or an imaging database) or an oncology information system. The oncology information system may provide treatment plan details to the treatment control system, such as received from treatment planning system. The treatment control system may use the treatment plan to control the particle treatment system (e.g., activate the gantry, the ion source, the accelerator, the scanning magnets, a particle beam, or the like). The treatment control system, for example, may include a beamlet intensity control, a beamlet energy control, a scanning magnet control, a table control, a gantry control, etc. In an example, the beamlet intensity control and the beamlet energy control may be used to activate a beamlet of a particular size or to target a particular location. The scanning magnetic control may be used to deliver beamlets according to the treatment plan, for example in a spiral pattern. The gantry control or the table control may be used to rotate the gantry.

The treatment planning software may include components such as a beamlet delivery and ordering component, with, for example, separate controls for beamlet ordering for spots or line segments. The treatment planning software is described in more detail above with respect to FIG. 18. The treatment planning software may access an imaging database to retrieve images or store information. When a treatment plan is completed, the treatment planning software may send the plan to an oncology information system for communication with the treatment control system.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

Method examples described herein may be machine or computer-implemented at least in part. Some examples may include a computer-readable medium or machine-readable medium encoded with instructions operable to configure an electronic device to perform methods as described in the above examples. An implementation of such methods may include code, such as microcode, assembly language code, a higher-level language code, or the like. Such code may include computer readable instructions for performing various methods. The code may form portions of computer program products. Further, in an example, the code may be tangibly stored on one or more volatile, non-transitory, or non-volatile tangible computer-readable media, such as during execution or at other times. Examples of these tangible computer-readable media may include, but are not limited to, hard disks, removable magnetic disks, removable optical disks (e.g., compact disks and digital video disks), magnetic cassettes, memory cards or sticks, random access memories (RAMs), read only memories (ROMs), and the like.

The invention is defined in the claims. Other embodiments, examples, items etc. are not a part of the invention.

## Claims

1. A computer-implemented method for protecting healthy tissue in particle arc therapy, the method comprising
defining a particle arc range for a radiotherapy treatment of a patient;
generating a spot selection for an arc sequence over the particle arc range, including a trajectory for delivering the radiotherapy treatment, based on a temporal dose heterogeneity parameter or a spatial dose heterogeneity parameter, wherein the temporal dose heterogeneity parameter or the spatial dose heterogeneity parameter includes temporal or spatial dose heterogeneity properties of irradiation of healthy tissue regions;
optimizing fluence of the arc sequence for the radiotherapy treatment; and
outputting the fluence optimized arc sequence for use in the radiotherapy treatment.

2. The method of claim 1, wherein the spot selection is generated based on both of the temporal dose heterogeneity parameter and the spatial dose heterogeneity parameter.

3. The method of claim 1, further comprising, optimizing a final spot trajectory of the fluence optimized arc sequence based on an improvement to delivery speed.

4. The method of claim 1, wherein selection of the temporal dose heterogeneity parameter minimizes a number of shots within non-targeted tissue.

5. The method of claim 4, wherein the number of shots represent a number of paints from different scanning layers or spot groups delivered within a specified time period on the non-targeted tissue.

6. The method of claim 5, wherein the spot groups include one or more of single spots, multiple spots within a single energy layer, single spots within multiple energy layers, or multiple spots within multiple energy layers and, optionally,
wherein the number of shots represent a number of paints from different scanning layers or spot groups delivered within a specified time period on the non-targeted tissue.

7. The method of claim 1, wherein selection of the spatial dose heterogeneity parameter maximizes a dose difference within a non-targeted tissue neighborhood.

8. The method of claim 7, wherein the maximized dose difference is measured in a number of paints by different spot groups having a minimum spatial distance of high dose regions within the non-targeted tissue neighborhood and, optionally, wherein the number of paints delivered to the non-targeted tissue neighborhood is minimized to zero or one.

9. The method of claim 2, wherein optimizing fluence of the arc sequence for the radiotherapy treatment is based on an applied temporal dose heterogeneity specific cost function and/or an applied spatial dose heterogeneity specific cost function.

10. A computer-implemented method for protecting healthy tissue in particle arc therapy, the method comprising
defining a particle arc range for a radiotherapy treatment of a patient;
generating an arc sequence for the particle arc range, including a trajectory for delivering the radiotherapy treatment;
optimizing fluence of the arc sequence for the radiotherapy treatment, based on an applied temporal dose heterogeneity specific cost function or an applied spatial dose heterogeneity specific cost function modifying the arc sequence, wherein the optimization includes temporal or spatial dose heterogeneity properties of irradiation of healthy tissue regions; and
outputting the fluence optimized arc sequence for use in the radiotherapy treatment.

11. The method of claim 10, wherein optimizing the fluence is based on both the applied temporal dose heterogeneity specific cost function and the applied spatial dose heterogeneity specific cost function, or
further comprising, optimizing final spot trajectory of the fluence optimized arc sequence based on an improvement to delivery efficiency.

12. The method of claim 10, wherein the applied temporal dose heterogeneity specific cost function minimizes a number of shots within non-targeted tissue.

13. The method of claim 12, wherein the number of shots represent a high dose overlap from different scanning layers or spot groups delivered within a specified time period on the healthy tissue and, optionally,
wherein minimizing the number of shots includes using a penalizing function, an additional objective, or a constraint.

14. The method of claim 12, wherein the number of shots represent a high dose overlap from different scanning layers or spot groups delivered within a specified time period on the non-targeted tissue.

15. The method of claim 10, wherein the applied spatial dose heterogeneity specific cost function maximizes a dose difference within a non-targeted tissue neighborhood.

16. A machine-readable medium including instructions that, when executed by processing circuitry, cause the processing circuitry to perform operations to implement any of the methods of claims 1 to 15.

17. An apparatus comprising means to implement any of the methods of claims 1 to 15.

## Patentansprüche

1. Computerimplementiertes Verfahren zum Schützen von gesundem Gewebe in der Particle-Arc-Therapie (PAT), wobei das Verfahren Folgendes umfasst:
Definieren eines Particle-Arc-Bereichs für eine Strahlentherapiebehandlung eines Patienten;
Generieren einer Punktauswahl für eine Bogensequenz über den Particle-Arc-Bereich, einschließlich einer Trajektorie zum Zuführen der Strahlentherapiebehandlung, basierend auf einem zeitlichen Dosisheterogenitätsparameter oder einem räumlichen Dosisheterogenitätsparameter, wobei der zeitliche Dosisheterogenitätsparameter oder der räumliche Dosisheterogenitätsparameter zeitliche oder räumliche Dosisheterogenitätseigenschaften der Bestrahlung gesunder Geweberegionen beinhaltet;
Optimieren der Fluenz der Bogensequenz für die Strahlentherapiebehandlung; und
Ausgabe der fluenzoptimierten Bogensequenz zur Verwendung in der Strahlentherapiebehandlung.

2. Verfahren nach Anspruch 1, wobei die Punktauswahl basierend auf sowohl dem zeitlichen Dosisheterogenitätsparameter als auch dem räumlichen Dosisheterogenitätsparameter generiert wird.

3. Verfahren nach Anspruch 1, ferner umfassend das Optimieren einer endgültigen Punkt-Trajektorie der fluenzoptimierten Bogensequenz basierend auf einer Verbesserung der Zuführgeschwindigkeit.

4. Verfahren nach Anspruch 1, wobei die Auswahl des zeitlichen Dosisheterogenitätsparameters eine Anzahl von Schüssen innerhalb von nicht-angezieltem Gewebe minimiert.

5. Verfahren nach Anspruch 4, wobei die Anzahl von Schüssen eine Anzahl von Überstreichungen aus verschiedenen Abtastschichten oder Punktgruppen repräsentiert, die innerhalb einer spezifizierten Zeitspanne auf dem nicht-angezielten Gewebe zugeführt werden.

6. Verfahren nach Anspruch 5, wobei die Punktgruppen eines oder mehrere von Einzelpunkten, mehreren Punkten innerhalb einer einzelnen Energieschicht, Einzelpunkten innerhalb mehrerer Energieschichten oder mehreren Punkten innerhalb mehrerer Energieschichten beinhalten, und optional
wobei die Anzahl von Schüssen eine Anzahl von Überstreichungen aus verschiedenen Abtastschichten oder Punktgruppen repräsentiert, die innerhalb einer spezifizierten Zeitspanne auf dem nicht-angezielten Gewebe zugeführt werden.

7. Verfahren nach Anspruch 1, wobei die Auswahl des räumlichen Dosisheterogenitätsparameters eine Dosisdifferenz innerhalb einer nicht-angezielten Gewebe-Nachbarschaft maximiert.

8. Verfahren nach Anspruch 7, wobei die maximierte Dosisdifferenz gemessen wird in einer Anzahl von Überstreichungen durch verschiedene Punktgruppen, aufweisend einen minimalen räumlichen Abstand von Hochdosisregionen innerhalb der nicht-angezielten Gewebe-Nachbarschaft, und optional wobei die Anzahl von Überstreichungen, die an die nicht-angezielte Gewebe-Nachbarschaft zugeführt werden, auf null oder eins minimiert wird.

9. Verfahren nach Anspruch 2, wobei das Optimieren der Fluenz der Bogensequenz für die Strahlentherapiebehandlung auf einer angewandten für zeitliche Dosisheterogenität spezifischen Kostenfunktion und/oder einer angewandten für räumliche Dosisheterogenität spezifischen Kostenfunktion basiert.

10. Computerimplementiertes Verfahren zum Schützen von gesundem Gewebe in der Particle-Arc-Therapie (PAT), wobei das Verfahren Folgendes umfasst:
Definieren eines Particle-Arc-Bereichs für eine Strahlentherapiebehandlung eines Patienten;
Generieren einer Bogensequenz für den Particle-Arc-Bereich, einschließlich einer Trajektorie zum Zuführen der Strahlentherapiebehandlung;
Optimieren der Fluenz der Bogensequenz für die Strahlentherapiebehandlung basierend auf einer angewandten für zeitliche Dosisheterogenität spezifischen Kostenfunktion oder einer angewandten für räumliche Dosisheterogenität spezifischen Kostenfunktion, was die Bogensequenz modifiziert, wobei die Optimierung zeitliche oder räumliche Dosisheterogenitätseigenschaften der Bestrahlung gesunder Geweberegionen beinhaltet; und
Ausgabe der fluenzoptimierten Bogensequenz zur Verwendung in der Strahlentherapiebehandlung.

11. Verfahren nach Anspruch 10, wobei das Optimieren der Fluenz sowohl auf der angewandten für zeitliche Dosisheterogenität spezifischen Kostenfunktion als auch der angewandten für räumliche Dosisheterogenität spezifischen Kostenfunktion basiert, oder
ferner umfassend das Optimieren der endgültigen Punkt-Trajektorie der fluenzoptimierten Bogensequenz basierend auf einer Verbesserung der Zuführungseffizienz.

12. Verfahren nach Anspruch 10, wobei die angewandte für zeitliche Dosisheterogenität spezifische Kostenfunktion eine Anzahl von Schüssen innerhalb von nicht-angezieltem Gewebe minimiert.

13. Verfahren nach Anspruch 12, wobei die Anzahl von Schüssen eine Hochdosis-Überlappung aus verschiedenen Abtastschichten oder Punktgruppen repräsentiert, die innerhalb einer spezifizierten Zeitspanne auf dem gesunden Gewebe zugeführt werden, und optional wobei das Minimieren der Anzahl von Schüssen das Verwenden einer Bestrafungsfunktion, eines zusätzlichen Ziels (Objektivs) oder einer Beschränkung beinhaltet.

14. Verfahren nach Anspruch 12, wobei die Anzahl von Schüssen eine Hochdosis-Überlappung aus verschiedenen Abtastschichten oder Punktgruppen repräsentiert, die innerhalb einer spezifizierten Zeitspanne auf dem nicht-angezielten Gewebe zugeführt werden.

15. Verfahren nach Anspruch 10, wobei die angewandte für räumliche Dosisheterogenität spezifische Kostenfunktion eine Dosisdifferenz innerhalb einer nicht-angezielten Gewebe-Nachbarschaft maximiert.

16. Maschinenlesbares Medium, das Anweisungen beinhaltet, die, wenn sie durch eine Verarbeitungsschaltungsanordnung ausgeführt werden, die Verarbeitungsschaltungsanordnung veranlassen, Operationen zum Implementieren beliebiger der Verfahren nach den Ansprüchen 1 bis 15 durchzuführen.

17. Vorrichtung, die Mittel zum Implementieren beliebiger der Verfahren nach den Ansprüchen 1 bis 15 umfasst.

## Revendications

1. Procédé mis en œuvre par un ordinateur, destiné à protéger le tissu sain dans une thérapie par faisceau de particules en arc, le procédé comprenant :
la définition d'une plage de portée des particules pour un traitement par radiothérapie d'un patient ;
la génération d'une sélection des spots pour une séquence d'arc sur la plage de portée des particules, comportant une trajectoire pour l'administration du traitement par radiothérapie, sur la base d'un paramètre d'hétérogénéité temporelle de dose ou d'un paramètre d'hétérogénéité spatiale de dose, le paramètre d'hétérogénéité temporelle de dose ou le paramètre d'hétérogénéité spatiale de dose comportant des propriétés d'hétérogénéité temporelle ou spatiale de dose d'irradiation de régions tissulaires saines ;
l'optimisation de la fluence de la séquence d'arc pour le traitement par radiothérapie ; et
l'émission de la séquence d'arc optimisée en fluence pour une utilisation dans le traitement par radiothérapie.

2. Procédé selon la revendication 1, la sélection des spots étant générée sur la base à la fois du paramètre d'hétérogénéité temporelle de dose et du paramètre d'hétérogénéité spatiale de dose.

3. Procédé selon la revendication 1, comprenant en outre l'optimisation d'une trajectoire finale de spots de la séquence d'arc optimisée en fluence sur la base d'une amélioration de la vitesse d'administration.

4. Procédé selon la revendication 1, la sélection du paramètre d'hétérogénéité temporelle de dose minimalisant un nombre de tirs dans le tissu non ciblé.

5. Procédé selon la revendication 4, le nombre de tirs représentant un nombre de « peintures » (« paints ») provenant de différentes couches de balayage ou de différents groupes de spots administrés dans une période de temps spécifiée sur le tissu non ciblé.

6. Procédé selon la revendication 5, les groupes de spots comportant un ou plusieurs éléments parmi des spots uniques, de multiples spots dans une seule couche d'énergie, des spots uniques dans de multiples couches d'énergie ou de multiples spots dans de multiples couches d'énergie et, éventuellement,
le nombre de tirs représentant un nombre de « peintures » (« paints ») provenant de différentes couches de balayage ou de différents groupes de spots administrés dans une période de temps spécifiée sur le tissu non ciblé.

7. Procédé selon la revendication 1, la sélection du paramètre d'hétérogénéité spatiale de dose maximisant une différence de dose dans un voisinage tissulaire non ciblé.

8. Procédé selon la revendication 7, la différence de dose maximisée étant mesurée dans un nombre de « peintures » (« paints ») par différents groupes de spots présentant une distance spatiale minimale par rapport à des régions de haute dose dans le voisinage tissulaire non ciblé et, éventuellement, le nombre de « peintures » (« paints ») administré au voisinage tissulaire non ciblé étant minimalisé à zéro ou un.

9. Procédé selon la revendication 2, l'optimisation de la fluence de la séquence d'arc pour le traitement par radiothérapie étant basée sur une fonction de coût spécifique d'hétérogénéité temporelle de dose appliquée et/ou une fonction de coût spécifique d'hétérogénéité spatiale de dose appliquée.

10. Procédé mis en œuvre par un ordinateur, destiné à protéger le tissu sain dans une thérapie par faisceau de particules en arc, le procédé comprenant :
la définition d'une plage de portée des particules pour un traitement par radiothérapie d'un patient ;
la génération d'une séquence d'arc pour la plage de portée des particules comportant une trajectoire pour l'administration du traitement par radiothérapie ;
l'optimisation de la fluence de la séquence d'arc pour le traitement par radiothérapie, sur la base d'une fonction de coût spécifique d'hétérogénéité temporelle de dose appliquée ou d'une fonction de coût spécifique d'hétérogénéité spatiale de dose appliquée modifiant la séquence d'arc, l'optimisation comportant des propriétés d'hétérogénéité spatiale de dose temporale ou spatiale d'irradiation de régions tissulaires saines ; et
l'émission de la séquence d'arc optimisée en fluence pour une utilisation dans le traitement par radiothérapie.

11. Procédé selon la revendication 10, l'optimisation de la fluence étant basée à la fois sur la fonction de coût spécifique d'hétérogénéité temporelle de dose appliquée et sur la fonction de coût spécifique d'hétérogénéité spatiale de dose appliquée ou
comprenant en outre l'optimisation d'une trajectoire finale de spots de la séquence d'arc optimisée en fluence sur la base d'une amélioration de l'efficacité d'administration.

12. Procédé selon la revendication 10, la fonction de coût spécifique d'hétérogénéité temporelle de dose appliquée minimalisant un nombre de tirs dans le tissu non ciblé.

13. Procédé selon la revendication 12, le nombre de tirs représentant un chevauchement de doses élevées provenant de différentes couches de balayage ou de différents groupes de spots administrées dans une période de temps spécifiée sur le tissu sain et, éventuellement,
la minimalisation du nombre de tirs comportant l'utilisation d'une fonction de pénalisation, d'un objectif supplémentaire ou d'une contrainte.

14. Procédé selon la revendication 12, le nombre de tirs représentant un chevauchement de doses élevées provenant de différentes couches de balayage ou de différents groupes de spots administrées dans une période de temps spécifiée sur le tissu non ciblé.

15. Procédé selon la revendication 10, la fonction de coût spécifique d'hétérogénéité spatiale de dose appliquée maximisant une différence de dose dans un voisinage tissulaire non ciblé.

16. Support lisible par machine comportant des instructions qui, lorsqu'elles sont exécutées par un circuit de traitement, amène le circuit de traitement à effectuer des opérations destinées à mettre en œuvre l'un quelconque des procédés selon les revendications 1 à 15.

17. Appareil comprenant des moyens destinés à mettre en œuvre l'un quelconque des procédés selon les revendications 1 à 15.
